(19) Europäisches Patentamt
European Patent Office
Office européen des brevets

(11) Publication number: **0 502 205 A1**

# (12) EUROPEAN PATENT APPLICATION published in accordance with Art. 158(3) EPC

(21) Application number: **91916220.6**

(22) Date of filing: **20.09.91**

(86) International application number: **PCT/JP91/01258**

(87) International publication number: **WO 92/05137 (02.04.92 92/08)**

(51) Int. Cl.[5]: **C07C 49/835**, C07C 49/84, C09K 3/00, A61K 7/42

(30) Priority: **21.09.90 JP 253726/90**

(43) Date of publication of application: **09.09.92 Bulletin 92/37**

(84) Designated Contracting States: **DE FR GB**

(71) Applicant: **NIPPON HYPOX LABORATORIES INCORPORATED**
**1759, Matsugaya Hachioji-shi**
**Tokyo 192-03(JP)**

(72) Inventor: **SATOH, Toshio, 57-3, Nagao, Joroku-cho**
**Tokushima-shi**
**Tokushima-ken 771-42(JP)**
Inventor: **MATSUMOTO, Hitoshi, 125-22, Shimofukuman**
**Hachiman-cho Tokushima-shi**
**Tokushima-ken 770(JP)**
Inventor: **MIKI, Tokutaro, 1759, Matsugaya Hachioji-shi**
**Tokyo 192-03(JP)**

(74) Representative: **Türk, Dietmar, Dr. rer. nat. et al**
**Türk, Gille + Hrabal Patentanwälte**
**Brucknerstrasse 20**
**W-4000 Düsseldorf 13(DE)**

## (54) CHALCONE DERIVATIVE AND ULTRAVIOLET SCREENING COSMETIC.

(57) Novel compounds represented by general formulae (I) and (II), and ultraviolet shielding cosmetic containing the same. In formula (I), $R^1$ represents hydrogen or hydroxyl, and $R^2$ represents $C_2$ or higher alkoxy, perfluorinated lower alkyl or fluorine. In formula (II), $R^5$ represents hydrogen or hydroxyl, and $R^6$ and $R^7$ represent each independently hydrogen, lower alkyl, lower alkoxy, perfluorinated lower alkyl, halogen, hydroxyl or nitro, or alternatively $R^6$ and $R^7$ are combined together to form a (a) group.

EP 0 502 205 A1

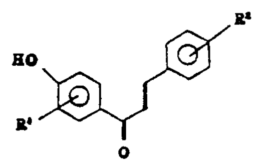

( I )

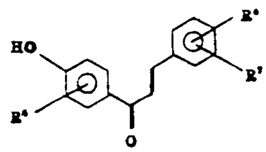

( II )

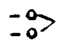

(a)

FIG. 2

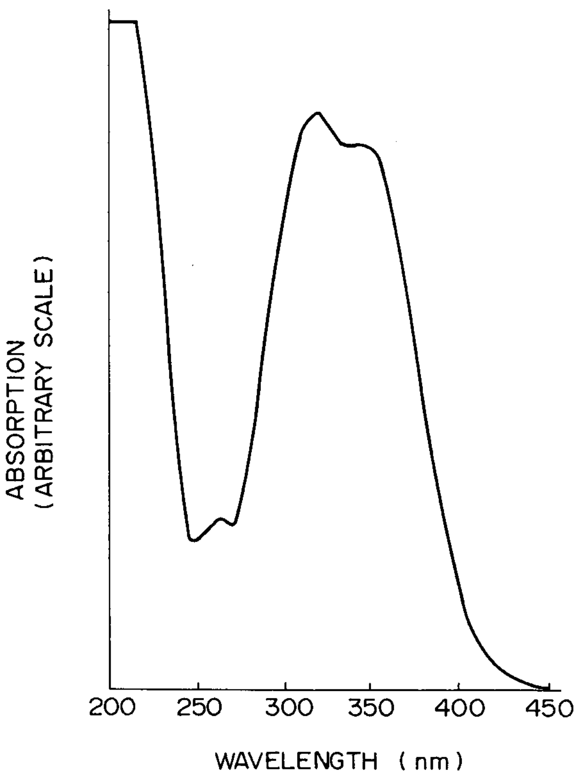
ABSORPTION (ARBITRARY SCALE)
200
250
300
350
400
450
WAVELENGTH (nm)

## TECHNICAL FIELD

The present invention relates to a chalcone derivative and an ultraviolet screening cosmetic.

## TECHNICAL BACKGROUND

In the skin exposed to direct sunlight or reflected light from snow for a long time, an increase in a melanin pigment (sunburn), erythema, blisters, etc., occur, and after the erythema formation, chromatosis is likely to occur. Further, skin stains and freckles are sometimes aggravated, and the skin aging is promoted. In some cases, a skin cancer occurs.

These changes in the skin are due to ultraviolet light contained in direct sunlight or reflected light from snow, particularly Dorno's ray having a wavelength of 280 to 315 nm and long-wavelength ultraviolet light having a wavelength of 315 to 400 nm. Therefore, a variety of cosmetics for protecting the skin from the harmful ultraviolet light have been conventionally developed and commercially available.

Recently, cosmetics used for the hair such as shampoos, hair rinses, etc., for protecting the hair from ultraviolet light have also been developed and commercially available.

These ultraviolet screening cosmetics can be largely classified into:

(1) cosmetics which contain an ultraviolet light absorber to absorb Dorno's ray and decrease the penetration of the Dorno's ray into the skin or the hair by means of this ultraviolet light absorber,

(2) cosmetics which contain an ultraviolet light absorber to absorb Dorno's ray and a light scattering agent (inorganic pigment) and decrease the penetration of Dorno's ray and long-wavelength ultraviolet light into the skin because the Dorno's ray is absorbed by the ultraviolet light absorber and the long-wavelength ultraviolet light is scattered by the light scattering agent, and

(3) cosmetics which contain an ultraviolet light absorber to absorb Dorno's ray and long-wavelength ultraviolet light and which decrease the penetration of the Dorno's ray and long-wavelength ultraviolet light into the skin or the air by means of the ultraviolet light absorber.

Meanwhile, not only an ultraviolet screening cosmetic but also a cosmetic of a direct skin-contact type is generally required not to cause inflammation and allergic reaction on the skin and not to cause rough skin.

For this reason, as an ultraviolet light absorber to be contained in an ultraviolet screening cosmetic, there is conventionally used an absorber which has low irritancy to the skin or is harmless to the skin.

However, the reaction of the skin to a cosmetic differs among individuals. There is a case where even a cosmetic which has low irritancy or is harmless to most of people causes inflammation, an allergic reaction or a rough skin on some people.

For example, p-aminobenzoic acid absorbs Dorno's ray having a wavelength of 270 to 300 nm and it is therefore conventionally used as an ultraviolet light absorber. It is, however, known that a cosmetic containing p-aminobenzoic acid as an ultraviolet light absorber, when used, causes contact dermatitis on some people.

Therefore, it is a first object of the present invention to provide a novel chalcone derivative which not only has an absorption band in most of the wavelength regions of Dorno's ray and long-wavelength ultraviolet light which are harmful ultraviolet light to the skin or the hair but also has high safety to a human body, and which is therefore useful as a component for an ultraviolet screening cosmetic.

Further, it is a second object of the present invention to provide an ultraviolet screening cosmetic which not only has an absorption band in most of the wavelength regions of Dorno's ray and long-wavelength ultraviolet light which are harmful ultraviolet light to the skin or the hair but also has high safety to a human body.

## DISCLOSURE OF THE INVENTION

The present inventors have found that chalcone derivatives of a certain type are suitable as an ultraviolet light absorber and at the same time excellent in safety to a human body, and the present invention has been accordingly completed.

That is, the chalcone derivative of the present invention to achieve the above first object comprises a compound represented by the following general formula (I),

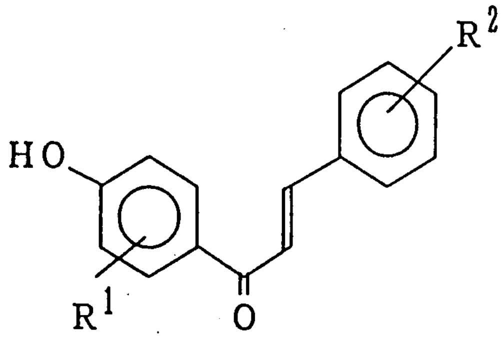

(I)

[wherein $R^1$ is a hydrogen atom or an OH group, and $R^2$ is a $C_2$ or higher alkoxy group, a perfluoro lower alkyl group or a fluorine atom],
or a salt thereof.

The cosmetic of the present invention to achieve the above second object characteristically contains a chalcone derivative comprising a compound represented by the following general formula (II),

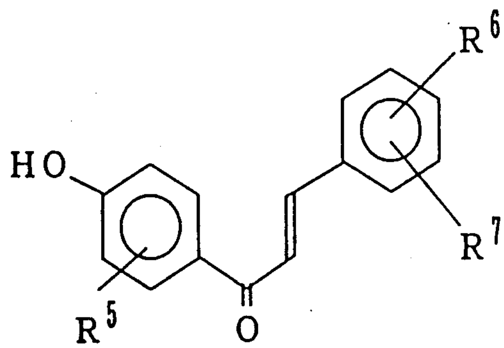

(II)

[wherein $R^5$ is a hydrogen atom or an OH group, each of $R^6$ and $R^7$ is, independently of other, a hydrogen atom, a lower alkyl group, a lower alkoxy group, a perfluoro lower alkyl group, a halogen atom, an OH group or a nitro group, or the combination of $R^6$ and $R^7$ represents a group of the following formula (III),

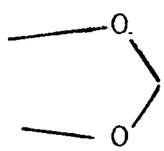

(III)

and/or a pharmacologically acceptable salt thereof.

BRIEF DESCRIPTION OF DRAWINGS

Fig. 1 is a graph showing the inhibition ratio of 2',4'-dihydroxychalcone which is one of chalcone derivatives used in the cosmetics according to the present invention to inflammation (swelling on a rat subplantar region) induced by an egg-white albumin-adjuvant system. Fig. 2 is a graph showing the ultraviolet absorption spectrum of 2',4'-dihydroxychalcone.

PREFERRED EMBODIMENTS FOR WORKING THE INVENTION

First, the chalcone derivative of the present invention will be explained. As described above, this chalcone derivative comprises a novel compound represented by the following general formula (I),

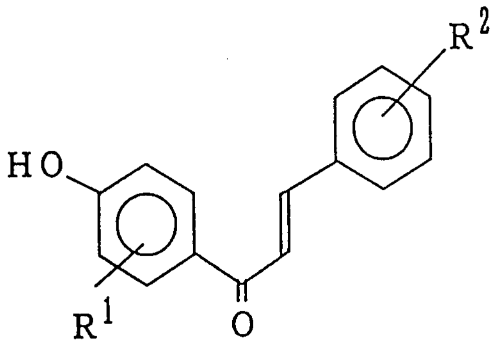

(I)

[wherein $R^1$ is a hydrogen atom or an OH group, and $R^2$ is a $C_2$ or higher alkoxy group, a perfluoro lower alkyl group or a fluorine atom],
or a salt thereof.

Specific examples of the $C_2$ or higher alkoxy group as $R^2$ include an ethoxy group, a propoxy group, a butoxy group, etc. Specific examples of the perfluoro lower alkyl group as $R^2$ include $-CF_3$, $-CF_2CF_3$, etc.

The novel compound represented by the above general formula (I) can be produced, for example, by allowing the following starting compounds (i) or (ii), which correspond to an intended compound,

(i) an acetophenone derivative having at least one tetrahydropyranyl group and a benzaldehyde or a derivative thereof, or

(ii) an acetophenone derivative having at least one tetrahydropyranyl group and a benzaldehyde derivative having at least one tetrahydropyranyl group,

to react in an alcoholic reaction solvent in the presence of an alkaline earth metal hydrate, and then either hydrolyzing the tetrapyranyl group contained in the reaction product in the presence of an acid, or subjecting it to alcoholysis in the presence of an acid (Chem. Abstr. Vol. 113, No. 25, 2309634).

All of chalcone derivatives comprising the novel compounds of the above general formula (I) which can be produced by the above method, etc., have an absorption band in most of wavelength regions of Dorno's ray and long-wavelength ultraviolet light. Further, these chalcone derivatives are all highly safe to a human body. Therefore, these chalcone derivatives are useful as a component for ultraviolet screening cosmetics, etc.

Further, chalcone derivatives comprising salts (sodium salt and potassium salt) of compounds represented by the above general formula (I) also have ultraviolet light absorption characteristics and safety to a human body which are similar to those of the chalcone derivatives comprising the compound represented by the general formula (I). Therefore, these salts are also useful as a component for ultraviolet screening cosmetics, etc. In addition, these salts can be produced by a conventional method.

Second, the ultraviolet screening cosmetic of the present invention will be explained.

As previously described, this cosmetic contains a compound represented by the following general formula (II),

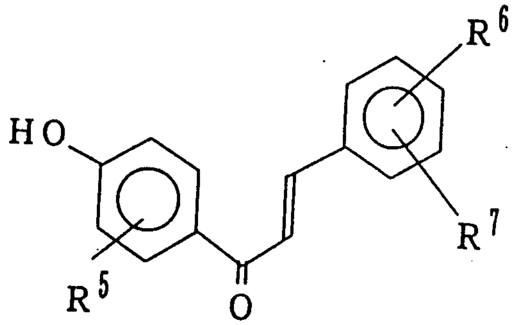

(II)

[wherein $R^5$ is a hydrogen atom or an OH group, each of $R^6$ and $R^7$ is, independently of other, a hydrogen atom, a lower alkyl group, a lower alkoxy group, a perfluoro lower alkyl group, a halogen atom, an OH group or a nitro group, or the combination of $R^6$ and $R^7$ represents a group of the following formula (III)
,

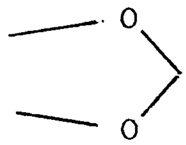

(III)

and/or a pharmacologically acceptable salt thereof.

Specific examples of the lower alkyl group as $R^6$ and/or $R^7$ include a methyl group, an ethyl group, an n-propyl group, an iso-propyl group, an n-butyl group, an iso-butyl group, a tert-butyl group, etc. Specific examples of the lower alkoxy group as $R^6$ and/or $R^7$ include a methoxy group, an ethoxy group, a propoxy group and a butoxy group. Specific examples of the perfluoro lower alkyl group as $R^6$ and/or $R^7$ include a $-CF_3$ group, a $-CF_2CF_3$ group, etc. And, specific examples of the halogen atom as $R^6$ and/or $R^7$ include a fluorine atom, a chlorine atom, a bromine atom, an iodine atom, etc.

The above general formula (II) includes the above-described novel compound of the general formula (I) in addition to known compounds. Specific examples of the novel compounds included in the above general formula (II) include those in which $R^5$, $R^6$ and $R^7$ are defined by the following (A) to (G).

(A) $R^5$ = 2'-OH group, $R^6$ = 3-fluorine atom, $R^7$ = hydrogen atom,
(B) $R^5$ = hydrogen atom, $R^6$ = 3-fluorine atom, $R^7$ = hydrogen atom,
(C) $R^5$ = 2'-OH group, $R^6$ = 2-fluorine atom, $R^7$ = hydrogen atom,
(D) $R^5$ = 2'-OH group, $R^6$ = 4-fluorine atom, $R^7$ = hydrogen atom,
(E) $R^5$ = 2'-OH group, $R^6$ = 4-$CF_3$ group, $R^7$ = hydrogen atom,
(F) $R^5$ = 2'-OH group, $R^6$ = 4-ethoxy group, $R^7$ = hydrogen atom,
(G) $R^5$ = 2'-OH group, $R^6$ = 2-ethoxy group, $R^7$ = hydrogen atom.

Further, among the compounds represented by the above general formula (II), those in which $R^5$, $R^6$ and $R^7$ are defined by the following (a) to (g) are known chalcone derivatives.

(a) $R^5$ = 2'-OH group, $R^6$ = $R^7$ = hydrogen atom,
(b) $R^5$ = hydrogen atom, $R^6$ = $R^7$ = hydrogen atom,
(c) $R^5$ = 2'-OH group, $R^6$ = 4-methyl group, $R^7$ = hydrogen atom,
(d) $R^5$ = 2'-OH group, $R^6$ = 4-chlorine atom, $R^7$ = hydrogen atom,
(e) $R^5$ = 2'-OH group, $R^6$ = nitro atom, $R^7$ = hydrogen atom,
(f) $R^5$ = hydrogen atom, the combination of $R^6$ and $R^7$ represents the group of the aforementioned formula (III),
(g) $R^5$ = hydrogen atom, $R^6$ = 3-OH group, $R^7$ = 4-OH group.

According to a study of the present inventors, the compounds of the above general formula (II) are found to have an absorption band in most of the wavelength regions of Dorno's ray and long-wavelength ultraviolet light. For example, as shown in Fig. 2 (ultraviolet absorption spectrum), 2',4'-dihdyroxychalcone which is a known chalcone derivative of the above (1) has an absorption band in most of the wavelength regions of Dorno's ray and long-wavelength ultraviolet light which are ultraviolet light harmful to the skin or the hair, and further, it absorbs Dorno's ray and long-wavelength ultraviolet light in a well-balanced way.

A study of the present inventors has also showed that the compounds represented by the above general formula (II) have pharmacological activities such as anti-inflammatory activity, anti-allergic activity, cell-protection activity, etc., and have high safety to a human body.

Further, all of the compounds represented by the above general formula (II) can be easily incorporated, according to a known method, into bases for a variety of cosmetics including foundation cosmetics such as cream, cake, milky lotion, lotion, etc., make-up cosmetics such as a lipstick, eye shadow, etc., medicated cosmetics such as products for the prevention of sunburn, a deodorant cosmetic, etc., cosmetics for the air such as a shampoo, a hair rinse, etc., cosmetics for a mouth, bath cosmetics such as a soap, bath liquid, etc., and the like.

Pharmacologically acceptable salts (sodium salt and potassium salt) of the compound of the above general formula (II) have the above-described ultraviolet light absorption characteristics and pharmacological activities as well, and can be incorporated into bases for a variety of cosmetics according to a known method. In addition, these salts can be obtained by a conventional method.

The cosmetic of the present invention contains a chalcone derivative having the above-described properties, i.e., a chalcone derivative comprising the compound represented by the above general formula (II) and/or a salt thereof. The preparation of the cosmetic is not specially limited, and there may be employed various preparations depending upon use thereof such as a variety of foundation cosmetics, a variety of make-up cosmetics, a variety of medicated cosmetics, a variety of cosmetics for the hair, a variety of cosmetics for the mouth, a variety of bath cosmetics, etc. The cosmetic according to any one of

these preparations can be easily produced by a conventional method. In some preparations, other ultraviolet light absorber and other light scattering agent may be used in combination.

The cosmetic of the present invention contains one or a plurality of chalcone derivatives comprising the compounds represented by the above general formula (II) and/or salts thereof. In this case, the content (total amount) of the chalcone derivatives is not specially limited, and can be set in the range of 0.01 to 99 wt.% depending upon intended use of the cosmetic. The content is particularly preferably 0.1 to 10 wt.%.

In addition, in the cosmetic of the present invention, the ultraviolet screening effect of the chalcone derivatives comprising the compound represented by the above general formula (II) and/or a salt thereof is not always required to be rated as the most preferred effect, but the pharmacological effect of the chalcone derivatives may be rated as the most preferred effect.

That is, as is clear from the above-described preparation examples, the ultraviolet screening cosmetic of the present invention is not necessarily limited to cosmetics for the protection of the skin or the hair from ultraviolet light. And, the pharmacological effect of the chalcone derivatives may be rated as the most preferred effect, and it may be used as a cosmetic for the mouth, a bath cosmetic or a cleansing cosmetic, one of foundation cosmetics. In this case, when the content (total amount) of the chalcone derivative(s) is set at 0.1 to 10 wt.%, the desired pharmacological effect can be obtained.

The chalcone derivative represented by the above general formula (II) can be produced not only by the previously described known method but also by condensing the starting materials in the presence of an alkali while acetophenone such as 2',4'-dihydroxyacetophenone, etc., is protected with a proper protecting group, and then removing the protecting group by a proper method. As the protecting group in this case, an acyl group, an alkoxyalkyl group, etc., may be used. Further, it can be produced by directly condensing an acetophenone derivative and a benzaldehyde derivative in the presence of an acid or an alkali without protecting it.

Examples of the present invention will be described hereinafter.

Example 1 [Preparation of 2',4'-dihydroxy-3-fluoro-chalcone (novel compound of the general formula (I))]

6.08 Grams of 2',4'-dihydroxyacetophenone and 0.36 g of p-toluenesulfonic acid pyridine salt were added to 100 ml of methylene chloride, and 5.05 g of 3,4-dihydro-2H-pyran was slowly added thereto. The mixture was stirred at 25 to 30° C for 3 hours, and the resultant reaction solution was consecutively washed with 5 % $NaHCO_3$, with 0.5N HCl and with 5 % $NaHCO_3$, and dried over anhydrous $Na_2SO_4$. Then, the solvent was distilled off.

5.0 Grams of m-fluorobenzaldehyde, 9.16 g of barium hydroxide $8H_2O$ salt and 100 ml of methanol were added to the above-obtained residue, and the mixture was stirred at 40° C for 10 hours. Added to the reaction solution was 500 ml of methylene chloride, and the mixture was consecutively washed with 1N HCl and with $H_2O$. Then, an organic layer was separated and recovered. This organic layer was concentrated under reduced pressure, and 200 ml of methanol was added to the resultant residue. The mixture was stirred for 1 hour to give a precipitate, and the precipitate was recovered by filtration.

This precipitate was added to 100 ml of an acetone solution of 0.5 % p-toluenesulfonic acid, and the mixture was stirred at room temperature for 3 hours. The reaction solution was concentrated under reduced pressure, and the resultant residue was recrystallized from 50 % acetone to give 2.62 g of the captioned compound.

Table 1 shows the melting point (mp), ultraviolet light absorption maximum ($UV_{max}$) and ultraviolet light absorbance of the so-obtained 2',4'-dihydroxychalcone.

Example 2 - Example 13

Example 1 was repeated except that the kinds of the acetophenone derivative and the benzaldehyde derivative were changed to give chalcone derivatives shown in Table 1 [novel compounds of the general formula (I): Example 2 - Example 7 and known compounds of the general formula (II): Example 8 - Example 13].

Table 1 or Table 2 shows the melting point (mp), ultraviolet light absorption maximum ($UV_{max}$) and ultraviolet light absorbance of each chalcone derivative.

Example 14

(1) Preparation of 2',4'-dihydroxychalcone (compound of the general formula (II): known chalcone derivative)

6.08 Grams of 2',4'-dihydroxyacetophenone and 0.36 g of p-toluenesulfonic acid pyridine salt were added to 100 ml of methylene chloride, and 5.05 g of 3,4-dihydro-2H-pyran was slowly added thereto. The mixture was stirred at 25 to 30° C for 3 hours, and the resultant reaction solution was consecutively washed with 5 % $NaHCO_3$, with 0.5N HCl and with 5 % $NaHCO_3$, and dried over anhydrous $Na_2SO_4$. Then, the solvent was distilled off.

4.24 Grams of benzaldehyde, 9.16 g of barium hydroxide $8H_2O$ salt and 100 ml of methanol were added to the above-obtained residue, and the mixture was stirred at 40° C for 10 hours. Added to the reaction solution was 500 ml of methylene chloride, and the mixture was consecutively washed with 1N HCl and with $H_2O$. Then, an organic layer was separated and recovered. This organic layer was concentrated under reduced pressure, and 200 ml of methanol was added to the resultant residue. The mixture was stirred for 1 hour to give a precipitate, and the precipitate was recovered by filtration.

This precipitate was added to 100 ml of an acetone solution of 0.5 % p-toluenesulfonic acid, and the mixture was stirred at room temperature for 3 hours. The reaction solution was concentrated under reduced pressure, and the resultant residue was recrystallized from 50 % acetone to give 3.02 g of the captioned compound.

Table 2 shows the melting point (mp), ultraviolet light absorption maximum ($UV_{max}$) and ultraviolet light absorbance of the so-obtained 2',4'-dihydroxychalcone.

(2) Preparation of ultraviolet screening cosmetic

A hydrophilic ointment of the Japanese Pharmacopoeia was used as a base. A small amount of this base and 1 g of the 2',4'-dihydroxychalcone obtained in Example 14(1) were fully kneaded together. The remaining base was added, and the mixture was fully kneaded to form an entirely homogeneous mixture of which the total amount was 100 g, whereby an ointment containing 1 wt.% of 2',4'-dihydroxychalcone was obtained.

Table 1

| | Formula | mp (°C) | $UV_{max}$ (nm) | Absorbance* 300 nm | 350 nm | $UV_{max}$ |
|---|---|---|---|---|---|---|
| Ex.1 | 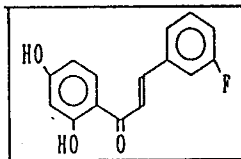 | 183~185 | 317 | 0.70~0.85 | 0.70~0.85 | 0.80~0.95 |
| Ex.2 | 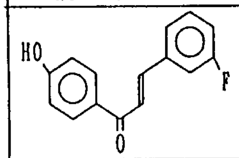 | 143~145 | 318 | 0.90~1.00 | 0.45~0.55 | 0.90~1.20 |
| Ex.3 | 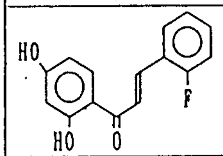 | 152~153 | 320 | 0.70~0.85 | 0.80~0.95 | 0.80~1.10 |
| Ex.4 | 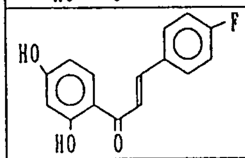 | 192~193 | 346 | 0.50~0.65 | 0.60~0.70 | 0.55~0.70 |
| Ex.5 | 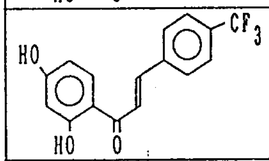 | 167~168 | 305 | 0.60~0.75 | 0.50~0.65 | 0.60~0.80 |
| Ex.6 | 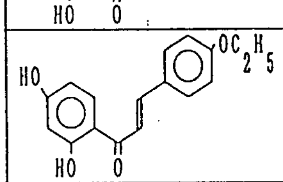 | 176~178 | 364 | 0.20~0.40 | 0.60~0.75 | 0.80~0.95 |
| Ex.7 | 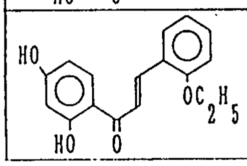 | 188~190 | 365 | 0.55~0.70 | 0.85~1.10 | 1.10~1.30 |

* Absorbance in a concentration of 10 μg/ml in an ethanol solution

Table 2

| | Formula | mp (°C) | $UV_{max}$ (nm) | Absorbance* 300 nm | 350 nm | $UV_{max}$ |
|---|---|---|---|---|---|---|
| Ex.8 | 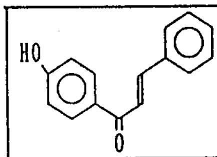 | 178~179 | 318 | 1.40~1.50 | 0.75~0.85 | 1.80~2.10 |
| Ex.9 | 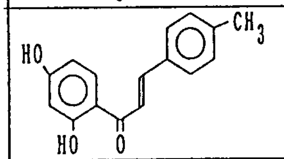 | 183~185 | 352 | 0.40~0.55 | 0.90~1.00 | 0.80~1.10 |
| Ex.10 | 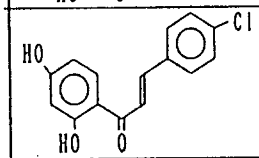 | 203~204 | 322 | 0.85~1.00 | 1.15~1.30 | 1.20~1.40 |
| Ex.11 | 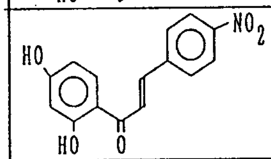 | 254~255 | 320 | 0.60~0.75 | 0.75~0.90 | 0.80~0.90 |
| Ex.12 | 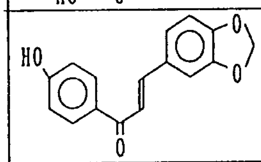 | 207~208 | 355 | 0.30~0.45 | 0.85~1.00 | 0.90~1.00 |
| Ex.13 | 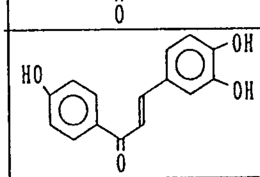 | 211~213 | 365 | 0.35~0.50 | 0.85~1.00 | 1.00~1.15 |
| Ex.14 | 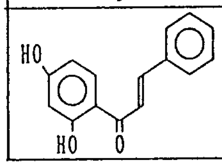 | 140~141 | 306 | 0.65~0.75 | 0.60~0.70 | 0.70~0.80 |

* Absorbance in a concentration of 10 μg/ml in an ethanol solution

Example 15

Example 14(2) was repeated with respect to each of the novel chalcone derivatives obtained in Example 1, Example 2, Example 4 and Example 6 and the known chalcone derivatives obtained in Example 8, Example 9 and Example 12 to give ointments containing 1 wt.% of the chalcone derivative each.

Example 16

Example 15 was repeated except that the content of each chalcone derivative was changed to 5 wt.% to give 7 ointments.

Example 17

Wilson paste was used as a base. A small amount of the base and 1 g of a novel chalcone derivative obtained in the same manner as in Example 1 were fully kneaded together. The remaining base was added, and the mixture was fully kneaded to form an entirely homogeneous mixture of which the total amount was 100 g, whereby a dermatologic paste containing 1 wt.% of the chalcone derivative of Example 1 was obtained.

The known chalcone derivatives obtained in Example 8, Example 12 and Example 14 were also treated in the same manner as above to obtain dermatologic pastes.

Example 18

A cream for the prevention of sunburn was prepared, by a conventional method, from 1.5 g of a novel chalcone derivative obtained in the same manner as in Example 1, 15.0 g of sesame oil, 3.0 g of hydrous lanolin, 25.0 g of a water-absorbing ointment of the Japanese Pharmacopoeia, 55.5 ml of purified water, a proper amount of an antioxidant and a proper amount of a flavor.

Further, the above procedures were also repeated with respect to each of the known chalcone derivatives obtained in Example 6, Example 12 and Example 14 to give creams for the prevention of sunburn.

Example 19

3 Grams of glycerin monostearate was melted over a water bath under heat, and 25 g of warmed glycerin, 1 g of methyl cellulose and 61 ml of purified water were added. While the mixture was stirred, 10 g of a novel chalcone derivative obtained in the same manner as in Example 1 was added. The mixture was stirred to form an entirely homogeneous mixture, whereby a suspension lotion was produced.

Further, the above procedures were repeated with respect to the known chalcone derivatives obtained in Example 8, Example 12 and Example 14 to give suspension lotions.

Ultraviolet screening test

10 Milligrams of the ointment containing 1 wt.% of the known chalcone derivative in Example 14, obtained in Example 15, 10 mg of an ointment containing 1 wt.% of p-aminobenzoic acid as a control, obtained in the same manner as in Example 15 except for the use of p-aminobenzoic acid in place of the above known chalcone derivative (to be abbreviated as AO in Table 3 which will follow), and 10 mg of the same hydrophilic ointment as that used in Example 15 (to be abbreviated as HO in Table 2 which will follow) were respectively applied to the femoral region of each of normally healthy persons (10 persons) such that these became circular forms having nearly the same size. Then, these applied portions were exposed to direct sunlight outdoors in fine weather for 2 hours and 30 minutes.

After the exposure, each applied portion was visually observed. The portions where clear sunburn was recognized were taken as +, the portions where slight sunburn was recognized were taken as ±, and the portions where almost no change was recognized were taken as -. The test substances were so evaluated on their ultraviolet screening effects. Table 3 shows the results.

The ointment containing 1 wt.% of the novel chalcone derivative of Example 1, obtained in Example 15, the ointment containing 1 wt.% of the novel chalcone derivative of Example 1, the ointment containing 1 wt.% of the novel chalcone derivative of Example 2, the ointment containing 1 wt.% of the novel chalcone derivative of Example 4, the ointment containing 1 wt.% of the novel chalcone derivative of Example 6 and the ointment containing 1 wt.% of the known chalcone derivative of Example 9 were respectively similarly tested on their ultraviolet screening effects. However, the exposure time outdoors in fine weather was set for 1 hour. Table 3 also shows the results.

Table 3

| | + | - | + |
|---|---|---|---|
| Known chalcone derivative of Example 14 | 0 | 1 | 9 |
| Novel chalcone derivative of Example 1 | 0 | 0 | 4 |
| Novel chalcone derivative of Example 2 | 0 | 0 | 4 |
| Novel chalcone derivative of Example 4 | 0 | 0 | 4 |
| Novel chalcone derivative of Example 6 | 0 | 0 | 3 |
| Known chalcone derivative of Example 9 | 0 | 0 | 4 |
| AO | 0 | 7 | 3 |
| HO | 9 | 1 | 0 |

*: Figures in columns show numbers of persons, and +, - and ± respectively have the following meanings.
+ ... Clear sunburn was recognized.
- ... Slight sunburn was recognized.
± ... Almost no change

As is clear from Table 3, the ultraviolet screening cosmetic of the present invention has been recognized to have an excellent screening effect against Dorno's ray and long-wavelength ultraviolet light.

Safety test

10 milligrams of the ointment containing 5 wt.% of the known chalcone derivative of Example 14, obtained in Example 16, was applied to the notal skins of Hartley guinea pigs, and the applied portions were covered with an adhesive plaster for 24 hours or 72 hours.

Thereafter, the skin of the applied portion and the skin of a normal portion were histologically observed and compared to show no difference between these two portions.

Further, 10 mg of the ointment containing 5 wt.% of the known chalcone derivative of Example 1, obtained in Example 16, was similarly tested to show no histological difference between the skin of the applied portion and the skin of a normal portion.

These test results show that the ultraviolet screening cosmetic of the present invention is excellent in the safety required of a cosmetic.

Anti-inflammatory activity test

The inhibition activity of the known chalcone derivative (2',4'-dihydroxychalcone) of Example 14 against inflammation induced by an egg-white albumin-adjuvant system was studied in the following manner.

As a test group, a comparison group and a control group, 5 male SD rats weighing 180 to 220 g were used per group. Each rat was measured for a size (volume) of the subplantar region of the right hind paw.

Sodium hydroxide was added to an aluminum sulfate aqueous solution, and the resultant aluminum hydroxide was obtained by filtration, washed with water and dried at 105° C for 2 hours. This aluminum hydroxide was used as an adjuvant. Per rat, a solution obtained by suspending 4 mg of the adjuvant and 0.2 mg of egg-white albumin in 0.1 ml of pertussis vaccine was prepared as an inflammation inducer.

Each rat of the test group was orally administered with a physiological saline solution containing 10 % Nikkol [Nikkol: trade name, dissolution aid, supplied by Nikko Chemical Co., Ltd.) containing 2',4'-dihydroxychalcone such that the dosage of 2',4'-dihydroxychalcone was 10 mg/weight-kg. 0.5 Hour after the administration, the inflammation inducer was intracutaneously administered into the subplantar region of the right hind paw of each rat to induce swelling.

Then, 0.5, 1, 2, 3 and 4 hours after the administration of the inflammation inducer, each rat was measured for a size (volume) of the subplantar region of the right hind paw by volumetry. Then, on the basis of the size (volume) of the subplantar region of the right hind paw of each rat measured before the administration of the 10 % Nikkol-containing physiological saline solution containing 2',4'-dihydroxychalcone, the swelling as a group in every measurement was determined by the following equation.

$$\text{Swelling (\%)} = \frac{Ps - Po}{Po} \times 100$$

Ps: Measurement value of volume of subplantar region at a measurement time (average of the group).

Po: Measurement value of volume of subplantar region before administration of inflammation inducer (average of the group).

Each rat of the control group was orally administered with a 10 % Nikkol-containing physiological saline solution in place of the 10 % Nikkol-containing physiological saline solution containing 2',4'-dihydroxychalcone. Thereafter, the same procedures as those for the test group were repeated to determine the swelling as a group.

Further, each rat of the comparison group was orally administered with a 10 % Nikkol-containing physiological saline solution containing a nonsteroidal anti-inflammatory drug, indomethacin, in place of the 10 % Nikkol-containing physiological saline solution containing 2',4'-dihydroxychalcone such that the dosage of indomethacin was 10 mg/weight kg. Thereafter, the same procedures as those for the test group were repeated to determine the swelling as a group.

Fig. 1 shows the results.

As is clear from Fig. 1, like indomethacin, 2',4'-dihydroxychalcone has significantly inhibited swelling and exhibited a clear anti-inflammatory activity when orally administered.

Anti-allergic activity test

A test of inhibition of free histamine from a rat abdominal cavity exudate mast cell used as an evaluation model for an anti-allergic activity was carried out in the following manner, and on the basis of this result, the anti-allergic activity of the known chalcone derivative (2',4'-dihydroxychalcone) of Example 14 was studied.

At first, an abdominal cavity exudate mast cell of an SD rat was obtained by a conventional method.

Then, the above-obtained abdominal cavity mast cell was floated on a Locke solution, and a test substance in a predetermined concentration and phosphatidylcerine (final concentration 3 µg/ml) were added. The mixture was warmed up to 37° C, and after concanavalin A as a freeing agent was added, the mixture was maintained at 37° C for 10 minutes.

Thereafter, the reaction solution was centrifugally separated to a sediment portion and a supernatant portion, and the histamine amount of each of these portions was quantitatively determined by an orthophthalaldehyde method.

In addition, as a test substance, a dimethylformamide solution (final concentration 0.03 mM) of 3 mM 2',4'-dihydroxychalcone and a DMF solution (final concentration 0.03 mM) of an anti-inflammatory drug (Azelastine) were used. The free histamine ratio was calculated on the basis of the following equation. In control, the solvent alone was added.

$$\text{Free histamine ratio (\%)} = \frac{Ps}{Ps + Pr} \times 100$$

Ps: Histamine amount in sediment portion

Pr: Histamine amount in supernatant portion.

The free histamine inhibition ratio of the test substance was calculated on the basis of the following equation.

$$\text{Inhibition ratio (\%)} = 100 - \frac{S - B}{C - B} \times 100$$

S: Free histamine ratio when test substance is added.

C: Free histamine ratio when no test substance is added (control).

B: Free histamine ratio when neither freeing agent nor test substance is added.

As a result, 2',4'-dihydroxychalcone showed an inhibition ratio of 56.9 ± 12.8 % (n = 5) when used in a concentration of 0.03 mM, and Azelastine used as a control in the same concentration showed an inhibition ratio of 20.8 ± 4.2 % (n = 5).

The above results have shown a strong anti-allergic activity of 2',4'-dihydroxychalcone.

Cell protection activity test

Cell impairment caused by a radical reaction of an organic compound is known as one of optical affections on the skin. Therefore, an inhibition test against a lipid peroxidation reaction of liver microsome which was a model of cell impairment caused through a radical reaction was carried out in the following manner, and on the basis of this result, the cell protection activity of various chalcone derivatives was studied.

First, rat liver microsome was obtained by a conventional method, and then, suspended in 1.15 % KCl to prepare a microsome suspension.

Then, the above microsome suspension in an amount of 2 mg as protein was added to a tris-HCl buffer (pH 7.4) containing NADPH (final concentration 0.2 mM), ADP (final concentration 1 mM) and $FeCl_3$ (final concentration 10 µM). And, 10 µl of a dimethylformamide (DMF) solution of a test compound was added so that the total amount was 1 ml, and the resultant mixture was warmed at 37° C for 20 minutes. The test compound was added such that the final concentration became $10^{-4}$ M or $10^{-5}$ M.

Thereafter, the amount of formed peroxidation lipid was measured by a thiobarbituric acid method. The inhibition activity of the test compound is expressed in terms of an inhibition ratio (%) in comparison with a control group. Table 4 shows the results. In addition, in the control group, 10 µl of DMF was used in place of 10 µl of the DMF solution of the test compound.

Table 4

| Kinds of chalcone derivatives | Inhibition ratio $10^{-4}$M concentration | $10^{-5}$ concentration |
|---|---|---|
| Novel Compounds | | |
| Example 1 | 90 % or more | 90 % or more |
| Example 2 | 90 % or more | 16 % |
| Example 3 | 90 % or more | 90 % or more |
| Example 4 | 90 % or more | 90 % or more |
| Example 5 | 90 % or more | 15 % |
| Example 6 | 90 % or more | 30 % |
| Example 7 | 90 % or more | 90 % or more |
| Known Compounds | | |
| Example 8 | 90 % or more | 64 % |
| Example 9 | 90 % or more | 25 % |
| Example 10 | 90 % or more | 32 % |
| Example 11 | 90 % or more | 62 % |
| Example 12 | 90 % or more | - |
| Example 13 | 90 % or more | 84 % |
| Example 14 | 90 % or more | 90 % or more |

*: n = 3 - 5

As is clear from Table 4, all the chalcone derivatives of the general formula (II) used in the cosmetic of the present invention significantly inhibit the lipid peroxidation reaction when used in a concentration of $10^{-4}$ M and/or when used in a concentration of $10^{-5}$ M. This data has clearly shown that the chalcone derivatives of the general formula (II) exhibit an effective cell protection activity against radical-inducing cell impairment.

As explained above, the chalcone derivative of the present invention has an absorption band in most of the wavelength regions of Dorno's ray and long-wavelength ultraviolet light, and it is highly safe to a human body since it has pharmacological activities such as anti-inflammatory activity, anti-allergic activity, cell

protection activity, etc. According to the present invention, therefore, there can be provided a novel chalcone derivative useful as a component for an ultraviolet screening cosmetic, etc.

Further, the cosmetic of the present invention has an absorption band in most of the wavelength region of ultraviolet light harmful to the skin or the hair, and has pharmacological activities such as anti-inflammatory activity, anti-allergic activity, cell protection activity, etc. According to the present invention, therefore, there can be provided an ultraviolet screening cosmetic which is highly safe to a human body.

**Claims**

1. A chalcone derivative comprising a compound represented by the following general formula (I),

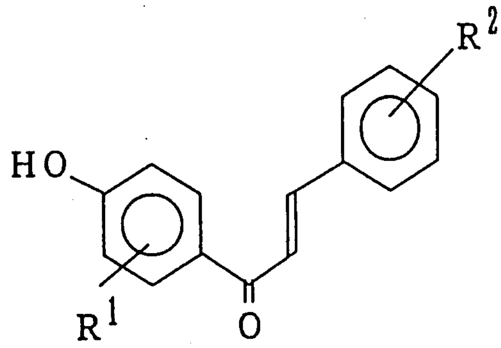

(I)

[wherein $R^1$ is a hydrogen atom or an OH group, and $R^2$ is a $C_2$ or higher alkoxy group, a perfluoro lower alkyl group or a fluorine atom],
or a salt thereof.

2. A chalcone derivative according to claim 1, wherein $R^2$ in the general formula (I) is an ethoxy group, a $-CF_3$ group or a fluorine atom.

3. An ultraviolet screening cosmetic containing a chalcone derivative comprising a compound represented by the following general formula (II),

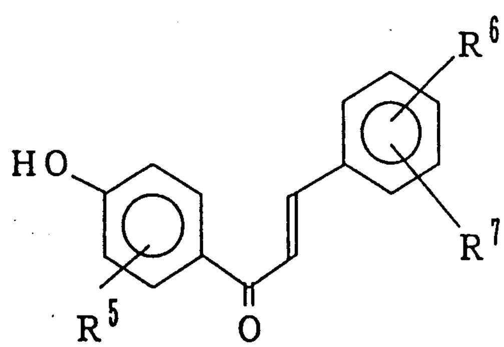

(II)

[wherein $R^5$ is a hydrogen atom or an OH group, each of $R^6$ and $R^7$ is, independently of other, a hydrogen atom, a lower alkyl group, a lower alkoxy group, a perfluoro lower alkyl group, a halogen atom, an OH group or a nitro group, or the combination of $R^6$ and $R^7$ represents a group of the following formula (III)],

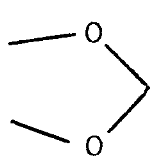

(III)

and/or a pharmacologically acceptable salt thereof.

4. A cosmetic according to claim 3, which contains a chalcone derivative comprising a compound of the general formula (II) in which $R^6$ in the is a $C_2$ or higher alkoxy group, a perfluoro lower alkyl group or a fluorine atom and $R^7$ is a hydrogen atom and/or a pharmacologically acceptable thereof.

5. A cosmetic according to claim 3, which absorbs Dorno's ray and/or long-wavelength ultraviolet light.

6. A cosmetic according to claim 3, which has at least one pharmacological activity selected from the group consisting of anti-inflammatory activity, anti-allergic activity and cell protection activity.

7. A cosmetic according to claim 3, wherein the chalcone derivative is contained in an amount of 0.01 to 99 wt.%.

8. A cosmetic according to claim 3, wherein the chalcone derivative is contained in an amount of 0.1 to 10 wt.%.

9. An ointment containing a chalcone derivative comprising a compound represented by the general formula (II) and/or a pharmacologically acceptable salt thereof.

10. A paste containing a chalcone derivative comprising a compound represented by the general formula (II) and/or a pharmacologically acceptable salt thereof.

11. A cream for the prevention of sunburn, which contains a chalcone derivative comprising a compound represented by the general formula (II) and/or a pharmacologically acceptable salt thereof.

12. A lotion containing a chalcone derivative comprising a compound represented by the general formula (II) and/or a pharmacologically acceptable salt thereof.

FIG. 1

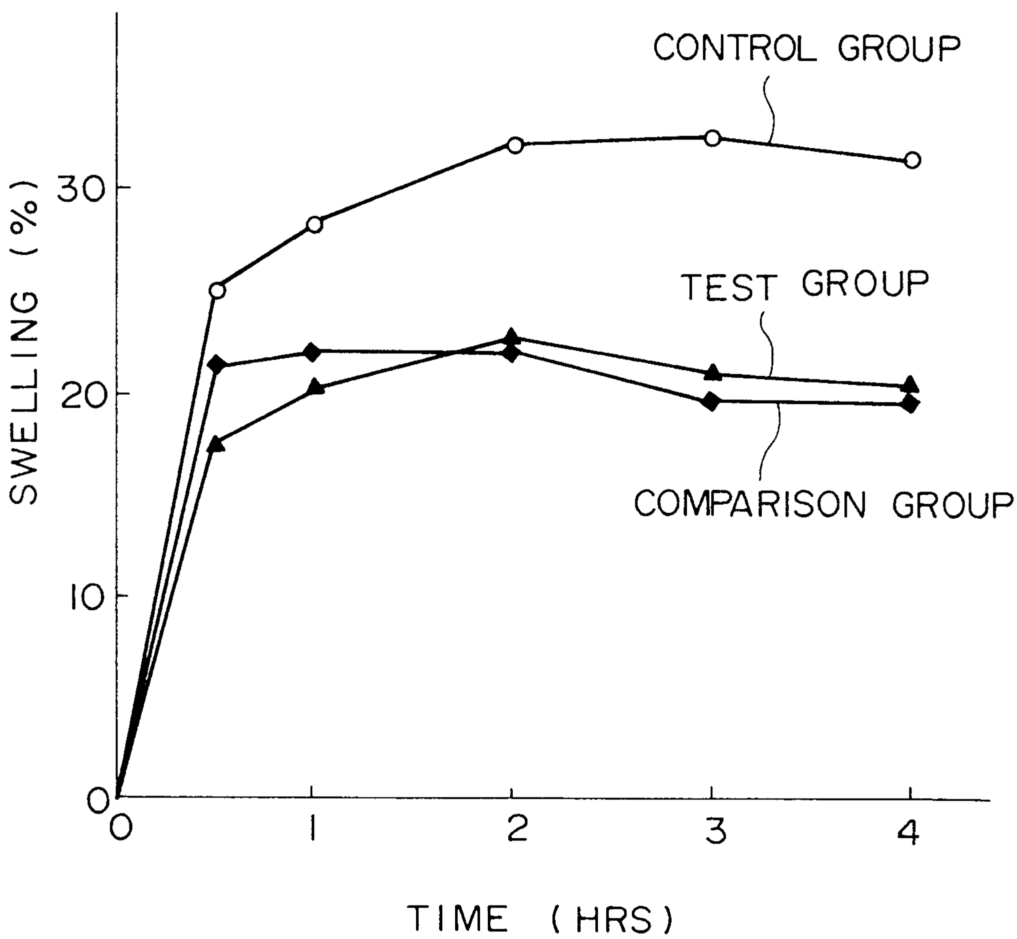
CONTROL GROUP
TEST GROUP
COMPARISON GROUP
SWELLING (%)
30
20
10
0
0
1
2
3
4
TIME (HRS)

FIG. 2

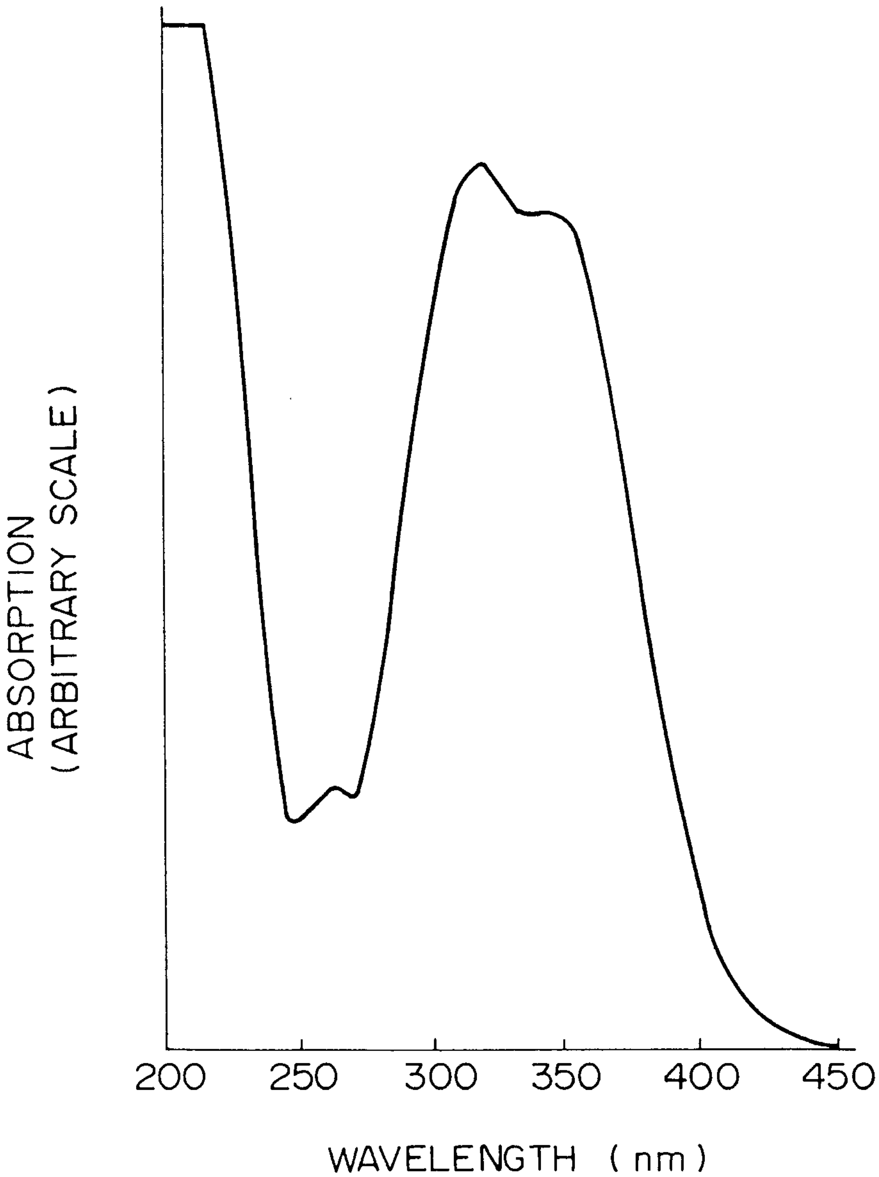
ABSORPTION
(ARBITRARY SCALE)
200
250
300
350
400
450
WAVELENGTH (nm)

# INTERNATIONAL SEARCH REPORT

International Application No PCT/JP91/01258

**I. CLASSIFICATION OF SUBJECT MATTER** (if several classification symbols apply, indicate all) [6]

According to International Patent Classification (IPC) or to both National Classification and IPC

Int. $Cl^5$ C07C49/835, 49/84, C09K3/00, A61K7/42

**II. FIELDS SEARCHED**

Minimum Documentation Searched [7]

| Classification System | Classification Symbols |
|---|---|
| IPC | C07C49/835, 49/84, C09K3/00, A61K7/42 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, B2, 64-1456 (Biorex Laboratories Ltd.), January 11, 1989 (11. 01. 89), & BE, A1, 864692 & DE, A1, 2810253 & FR, A1, 2383157 & ZA, A, 7800952 & ES, A1, 467961 & US, A, 4190671 & GB, A, 1566497 & AU, B2, 520288 & IT, A, 1113112 | 1, 2 |
| A | JP, A, 63-166848 (Matsuura Yakugyo K.K.), July 11, 1988 (11. 07. 88), (Family: none) | 3-12 |
| A | JP, A, 60-109544 (Kao Corp.), June 15, 1985 (15. 06. 85), & GB, A0, 8427998 & FR, A1, 2555167 & DE, A1, 3441636 & GB, A1, 2149789 & US, A, 4584190 | 3-12 |

* Special categories of cited documents: [10]

- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step
- "Y" document of particular relevance; the claimed invention cannot be considered to involve an inventive step when the document is combined with one or more other such documents, such combination being obvious to a person skilled in the art
- "&" document member of the same patent family

**IV. CERTIFICATION**

| Date of the Actual Completion of the International Search | Date of Mailing of this International Search Report |
|---|---|
| November 8, 1991 (08. 11. 91) | November 25, 1991 (25. 11. 91) |
| **International Searching Authority** | **Signature of Authorized Officer** |
| Japanese Patent Office | |